(19) Europäisches Patentamt / European Patent Office / Office européen des brevets

(11) **EP 1 826 293 A1**

(12) **EUROPEAN PATENT APPLICATION**

(43) Date of publication:
29.08.2007 Bulletin 2007/35

(51) Int Cl.:
*C23F 4/00* (2006.01)  *A61F 2/30* (2006.01)
*A61L 27/06* (2006.01)

(21) Application number: 07002850.1

(22) Date of filing: 09.02.2007

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(30) Priority: 09.02.2006 US 771834 P

(71) Applicant: Isoflux, Inc.
Rochester, NY 14624 (US)

(72) Inventors:
• Glocker, David A.
West Henrietta, NY 14586 (US)
• Romach, Mark
Spencerport, NY 14559 (US)

(74) Representative: Kramer, Reinhold
Kramer - Barske - Schmidtchen
"Patenta"
Radeckestrasse 43
81245 München (DE)

(54) **Formation of nanoscale surfaces for the attachment of biological materials**

(57) An inhomogeneous surface is formed on a substrate (103), such as an orthopedic implant or other surface upon which cell growth is desired, by depositing a discontinuous coating of atoms (101) on the substrate (103) and etching the substrate (103). The difference in etch rates between the coating (101) and the substrate (103) will produce structures (106) in the nanometer scale. Deposition and etch conditions can be chosen to create structures (106) of a specific size to preferentially bind to specific biological materials.

Fig. 1

EP 1 826 293 A1

## Description

### FIELD OF THE INVENTION

The invention broadly relates to the modification of surfaces by deposition and etching, more specifically to modified surfaces having surface structures and inhomogeneities, and even more particularly to modified surfaces having surface structures and inhomogeneities arranged to promote the cell growth on and attachment to the modified surface.

### BACKGROUND OF THE INVENTION

Open, porous structures have been found to promote the attachment of natural tissue to an implanted device. Under proper conditions, some sintered powders can form such porous materials. For example, sintered powders of materials such as Tantalum (Ta), Titanium (Ti), Niobium (Nb), Chromium (Cr) and others, as well as their alloys, are often chosen for these applications because these materials are both corrosion resistant and biocompatible. Examples of applications of sintered powders are described in United States Patent Nos. 5,282,861; 5,669,909; 5,984,967; 6,261,322; 6,645,206; 6,613,091; and, 6,375,655. In addition to sintering material powders, other methods for producing porous structures are also known, e.g., filling a vitreous carbon matrix via chemical vapor deposition. Pores and surface features formed by both sintering material powders and filling vitreous carbon matrices by chemical vapor deposition have typical sizes, e.g., diameters and depths/heights, from fractions of a millimeter to micrometers. As used herein, such structures are referred to as mechanically rough.

Recently it has been found that some small surface features with sizes of approximately one hundred (100) nanometers promote the attachment of bone cells to metals (*See* R&D Magazine, January 2004, p46). Surface features having sizes from approximately ten (10) nanometers to approximately one hundred (100) nanometers mimic the texture of natural bone, and are also comparable to the size of proteins needed to promote cell growth. It is believed that the precise shape and pattern of these features is not critical to their usefulness, and thus they can be regular or irregular in shape. However, heretofore, the formation of such small features has been inconsistent, unreliable and/or expensive.

Medical implants have been designed in the past to permit large-scale ingrowth of tissue. For example, orthopedic implants such as artificial knees and hips, which are critical to improving the quality of life for millions of people each year, have been designed to incorporate mechanically rough features so that bone will attach to the implanted device. These surfaces allow bone to become incorporated into the mechanically rough features, but the surfaces do not of themselves stimulate the growth of bone. It becomes more critical that such implants are easily and repeatably manufactured because as the population ages, need for such implants will likely continue to increase, and for the use of such implants to be successful, the devices must be biologically accepted, i.e., bone and tissue must sufficiently bond to the implants.

Thus, there has been a long-felt need for methods to stimulate cell growth that can be formed directly on orthopedic implants and other devices in a simple and cost effective manner.

### SUMMARY OF THE INVENTION

The subject invention broadly comprises methods of modifying a surface to produce surface structures and inhomogeneities on a nanometer scale in order to promote cell growth on and/or attachment to the surface for various applications. Generally, the subject invention deals with physical vapor deposition and removal processes, such as sputtering, cathodic arc and thermal evaporation, to produce such nanometer scale surface structures.

In an embodiment, the invention broadly comprises a method of forming an inhomogeneous surface including the steps of: depositing a discontinuous coating of atoms of a first substance on a substrate comprising a second substance, said first and second substances having first and second etch rates, respectively, wherein said first etch rate is different than said second etch rate; and, etching the substrate. In some embodiments, the step of etching the substrate includes etching the substrate via physical sputtering, while in other embodiments, the step of depositing the discontinuous coating includes depositing via physical vapor deposition. In still other embodiments, the steps of depositing and etching are performed simultaneously.

In further embodiments, the discontinuous coating of atoms forms a plurality of clusters, each of the plurality of clusters having lateral dimensions from about ten nanometers to about one thousand nanometers. In yet further embodiments, the inhomogeneous surface includes a plurality of structures, each of the structures having heights from about ten nanometers to about ten thousand nanometers. In still further embodiments, the discontinuous coating is deposited on a mechanically rough portion of the substrate, and in some embodiments, the first etch rate is less than the second etch rate.

In other embodiments, the method further includes the step of: applying a voltage to the substrate, wherein applying the voltage causes a formation of structures on the substrate, each of the structures having a height of about one hundred nanometers to about ten thousand nanometers. In yet other embodiments, the discontinuous coating of atoms forms a plurality of clusters and dimensions of the plurality of clusters are arranged to create a plurality of final structures that preferentially bind at least one biological structure having a specific size. In some embodiments, the method further includes the step of: depositing a layer on the substrate, wherein the layer and the coating have a first chemical binding energy, the layer and the substrate have a second chemical binding energy, the first and second chemical binding energies are different, and the first and second chemical binding energies are arranged to alter nucleation characteristics of the coating. In several embodiments, the substrate is a part of an orthopedic implant.

The present invention may also broadly comprise a method of preparing a substrate for cell attachment comprising the steps of: providing the substrate; and, depositing a coating of atoms on the substrate so that the atoms form clusters, each of the clusters having a size and a distance from other of the clusters. In some embodiments, the size of each cluster and the distance from other of the clusters are chosen to preferentially bind at least one biological structure having a specific size. In other embodiments, the method further includes the step of: changing a chemical binding energy between the substrate and the coating of the atoms.

In a further embodiment, the present invention broadly comprises a method of creating a surface for enhancing cell growth on the surface, the surface includes a first substance, the method includes the steps of: depositing a discontinuous coating of atoms of a second substance on the surface, wherein the first and second substances have first and second sputter yields, respectively, and the first and second sputter yields are different; and applying a voltage to the surface to cause sputtering. In some embodiments, the steps of depositing the coating and applying the voltage are performed simultaneously, while in other embodiments, the step of applying the voltage is performed after the step of depositing the coating. In still further embodiments, the surface is a part of an orthopedic implant.

In yet a further embodiment, the present invention broadly comprises a substrate arranged for cell attachment including a surface and a discontinuous coating of atoms on the surface, wherein the coating includes a plurality of clusters of atoms, each of the plurality of clusters having lateral dimensions from about ten nanometers to about one thousand nanometers.

## BRIEF DESCRIPTION OF THE DRAWINGS

These and other features, aspects and advantages of the present invention will become better understood with regard to the following description, appended claims, and accompanying drawings where:

Figure 1 is a cross-sectional view of a substrate having a discontinuous coating of atoms; and,

Figure 2 is a cross-sectional view of the substrate of Fig. 1 after etching.

## DETAILED DESCRIPTION OF THE INVENTION

At the outset, it should be appreciated that like drawing numbers on different drawing views identify identical, or functionally similar, structural elements of the invention. While the present invention is described with respect to what is presently considered to be the preferred embodiments, it is to be understood that the invention as claimed is not limited to the disclosed embodiments.

Furthermore, it is understood that this invention is not limited to the particular methodology, materials and modifications described and as such may, of course, vary. It is also understood that the terminology used herein is for the purpose of describing particular embodiments only, and is not intended to limit the scope of the present invention, which is limited only by the appended claims.

Unless defined otherwise, all technical and scientific terms used herein have the same meaning as commonly understood to one of ordinary skill in the art to which this invention belongs. Although any methods, devices or materials similar or equivalent to those described herein can be used in the practice or testing of the invention, the preferred methods, devices, and materials are now described.

Ultra thin coatings deposited using physical vapor deposition, or in other words those layers having average thicknesses from less than a monolayer, *i.e.*, a single atomic layer, to tens of monolayers, do not ordinarily condense as a uniform coating. Rather, the atoms nucleate as clusters whose size and spacing are determined by such factors as substrate temperature, chemical binding energy between the coating and substrate, energy of the arriving atoms, *etc.* Therefore, the average height of these clusters may be significantly greater than the average thickness of the overall coating, while the regions between the clusters are merely bare substrate material. The instant invention makes use of differences in etch rates that can exist between such clusters and the underlying substrate material, in order to produce structures that have dimensions of tens to hundreds of nanometers in breadth and height in and on the substrate.

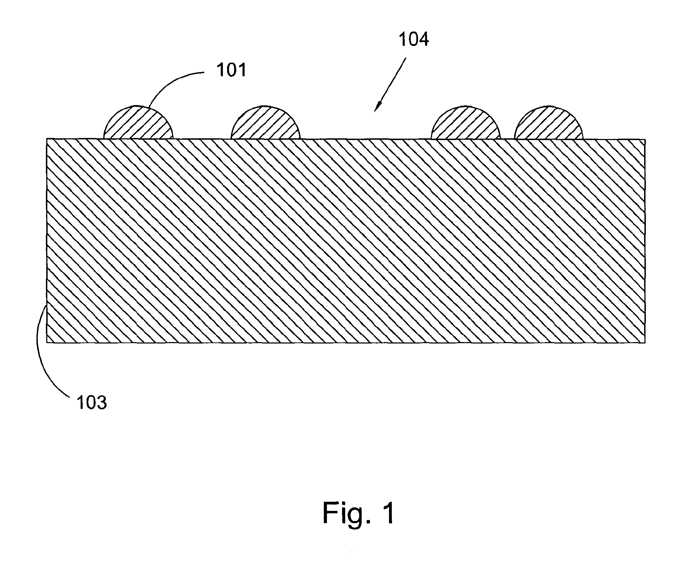

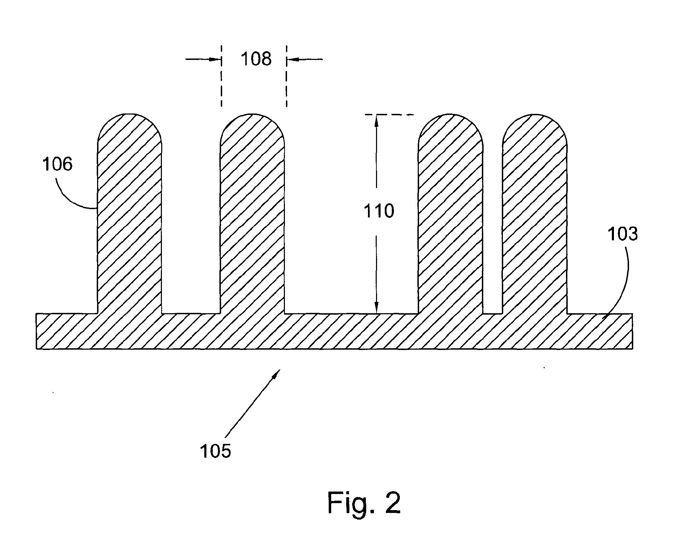

Adverting now to the figures, Figure 1 is a cross-sectional view of a substrate having a discontinuous coating of atoms, more specifically, a coating of aluminum oxide ($Al_2O_3$) clusters 101 randomly spaced about titanium substrate 103 thereby forming coated substrate 104, while Figure 2 is a cross-sectional view of coated substrate 104 after etching. The following discussion is perhaps best understood in view of both Figures 1 and 2. In the embodiment shown in the figures, Ti substrate 103 is used as a base layer upon which $Al_2O_3$ clusters 101 are deposited. $Al_2O_3$ clusters 101 are attached to Ti substrate 103 and approximately several nanometers in height and approximately several nanometers in diameter. Under ion bombardment, the sputter yield of $Al_2O_3$ clusters 101, *i.e.*, the number of $Al_2O_3$ atoms ejected from coated substrate 104 per incident ion, is approximately a few percent of that of the atoms ejected from Ti substrate 103. Thus, after depositing clusters 101 on Ti substrate 103, coated substrate 104 is subjected to ion bombardment to cause sputtering. Initially, coated substrate 104 will be etched only in those areas not covered by $Al_2O_3$ clusters 101. By continuing to etch coated substrate 104 until $Al_2O_3$ clusters 101 are removed, the resulting etched substrate 105 will have high aspect ratio structures 106 with spacings that reflect the original spacing of the $Al_2O_3$ clusters 101. Thus, Figure 2 shows the results of coating $Al_2O_3$ clusters 101 on Ti substrate 103 to form coated substrate 104, and the subsequent removal of $Al_2O_3$ clusters 101 by ion bombardment. It has been found that even if the substrate material, *e.g.*, Ti substrate 103, has a low sputter yield surface, such as a native oxide, removing that surface will require the same length of time in all locations. Therefore, the difference in sputter rates for the deposited clusters 101 and substrate 103 will still dictate the vertical size of the resulting structures 106. It should be noted that as used herein lateral dimension or diameter is used to refer to diameters 108, while vertical size, height and depth are used to refer to height 110.

Although coating a substrate with $Al_2O_3$ is described in the foregoing embodiment, one of ordinary skill in the art will recognize that a wide variety of coating materials may be used, *e.g.*, metals, oxides, nitrides and alloys, and such variations are within the spirit and scope of the claimed invention. However, it has been found that metal oxides such as $Al_2O_3$ as well as oxides of Titanium (Ti), Molybdenum (Mo), Niobium (Nb), Chromium (Cr) and others have very low sputter yields and are, therefore, particularly advantageous when used for coating a substrate. Such materials are good candidates for producing randomly spaced clusters of atoms on a nanometer scale, such as $Al_2O_3$ clusters 101. Hereinafter, such nanometer scale coatings are referred to as a "nanomask."

As those skilled in the art will appreciate, the nanomask, *e.g.*, $Al_2O_3$ clusters 101 may be deposited using a source of the mask material or may be deposited reactively by, for example, sputtering a metal in a chamber containing oxygen ($O_2$), nitrogen ($N_2$), or some other compound forming gas. Any number of well-known means, such as sputtering, cathodic arc evaporation, thermal evaporation and chemical vapor deposition can deposit discontinuous clusters 101. As mentioned previously, the deposition conditions strongly affect clusters 101 size and spacing, and conditions are chosen which produce the desired results.

For the purposes of bone growth, nucleation characteristics resulting in a discontinuous coating of clusters 101 having diameters from about several nanometers to about several hundreds of nanometers, and heights from about several nanometers to about several hundreds of nanometers, have been found to be particularly advantageous. The dimensions of resulting structures 106 of course still depend on the ratio of the etch rate of substrate 103 to the etch rate of clusters 101. Although the aforementioned embodiment is described in terms of preferentially bonding to bone, one of ordinary skill in the art will recognize that a substrate have clusters of different dimensions than previously set forth will preferentially bond to other types of cells, and such variations are within the spirit and scope of the claimed invention. In a preferred embodiment, resulting structures 106 have lateral dimensions, *i.e.*, diameters 108, from approximately ten (10) to several hundreds of nanometers across and heights 110 from approximately ten (10) to ten thousand (10,000) nanometers.

The height *H* of a given resulting structure 106 will be:

$$H = R \times h,$$

Where *h* is the height of the initial cluster 101 that produced structure 106 and *R* is the ratio of the etch rate of substrate 103 to the etch rate of cluster 101. Of course, a given cluster 101 will not have a single height, but will be domed or otherwise irregular, and therefore, the resulting structure 106 may also be irregularly shaped. For example, as is well known from published sputter yields for $Al_2O_3$ and Ti, an $Al_2O_3$ nanomask deposited on a Ti substrate and sputtered using 500 electron volts (eV) under Argon (Ar) will result in a ratio *R* of approximately 17. Therefore, if a nanomask cluster of atoms had a height h of 10 nanometers, the height *H* of the resulting structure would be approximately 170 nanometers.

In order to control the nucleation characteristics of the nanomask coating, it is possible to change the chemical binding energy between substrate 103 and the coating material, *e.g.*, $Al_2O_3$. For example, a very thin layer of a material having weak chemical bonding with the nanomask material, such as a hydrocarbon, may be deposited onto the substrate prior to the deposition of the coating material. Such a low energy coating, as it is known, will result in fewer, larger nuclei of the nanomask material, clusters 101. Alternatively, it is possible to use plasma cleaning as an integral part of the coating process to change the nucleation characteristics. In that case, an initial high voltage can be applied to substrate 103 in order to clean substrate 103 and remove any residual contamination. This cleaning may be done with the deposition source off or it may be carried out during the initial stages of deposition. Times for such cleaning may range from less than a minute to several minutes.

For purposes of cell attachment, coated substrate 104 may not require etching in order to form preferred sites for cell growth. In certain cases it is possible that material boundaries formed between substrate 103 and clusters 101 will produce enough of discontinuity in surface characteristics to stimulate the attachment of cells at the locations of clusters 101 and/or therebetween clusters 101. It has been found, for example, that material boundaries on such scales may result in relatively large local electric fields, which may enhance the attachment of biological materials at those locations. For example, a discontinuous coating of Gold (Au) on Ti may result in large chemical potentials at the boundaries of the two materials that stimulate biological materials, such as proteins, to locate preferentially at those boundaries. As one of ordinary skill in the art will appreciate, other types of dissimilar materials are also candidates for such nanoscale coating clusters, and such variations are within the scope of the claimed invention.

Clusters 101 may be deposited on otherwise smooth portions of substrate 103 or it is also possible to form clusters 101 on the surfaces of a sintered powder, thereby creating a surface with two roughness scales. In addition, if clusters 101 are porous they may be infused with bioactive materials, such as superoxide dismutuse to inhibit inflammation or proteins to promote bone growth.

As described *supra*, once clusters 101 are deposited on substrate 103, thereby forming coated substrate 104, structures 105 can be produced by etching coated substrate 104. Any etching known in the art may be used, such as reactive or non-reactive ion etching. For example, introducing an inert gas such as Argon at a pressure from approximately one (1) mTorr to one hundred (100) Torr, and applying a voltage to coated substrate 104 that is high enough to cause physical sputtering, typically between one hundred (100) and one thousand (1000) volts (V), will result in the desired etching. The sputtering voltage may be direct current (DC), pulsed DC, radio frequencies (RF) in the megahertz range, or an intermediate frequency, *i.e.*, alternating current (AC), and such voltage should be applied under conditions that produce a glow discharge. The gas used may be inert, such as Ar, or can be chosen to accentuate the difference in sputtering rates between clusters 101 and substrate 103. For example, if clusters 101 are a metal oxide and substrate 103 is a polymer, it is known in the art that a plasma containing $O_2$ will etch the polymer very quickly while etching the metal oxide slowly. Such a process is known as reactive ion etching and relies on chemical processes as well as physical bombardment to remove material.

The above described etching processes are common in the electronics industry, where etch masks are routinely used to produce specific desired patterns in integrated circuits, for example. However, in those cases the patterns that define the final structure are made using lithography, which is an expensive process. In the method of the instant invention, the patterns are formed on the surfaces of implantable devices by choosing deposition conditions that form a random pattern of clusters of atoms, and therefore is far more cost effective and simple to perform than lithography processes.

The deposition of clusters 101 and subsequent etching of coated substrate 104 may be done in one continuous operation, or may be performed sequentially. An example of a continuous operation is depositing $Al_2O_3$ clusters 101 onto Ti substrate 103 using RF sputtering. During deposition of clusters 101, a voltage may also be applied to substrate 103. The voltage should be kept low enough that it will not cause clusters 101 to be removed faster than they are deposited. However, once clusters 101 are properly deposited on substrate 103, the voltage may be increased to cause sputtering of both clusters 101 and substrate 103 in such a way that there is a net removal of material, and the formation of nanostructures 106 as described above. It has been found that using RF sputtering to deposit clusters 101 is a relatively inefficient deposition process. That is, a relatively intense RF plasma is needed to produce even a small deposition rate of a nanomask material such as $Al_2O_3$. However, because the nanomask material is so thin on average, a low deposition rate is often acceptable. The advantage of using RF sputtering arises once the nanomask is deposited. By leaving the RF power on and applying a DC voltage to coated substrate 104, the intense RF plasma provides a dense source of ions which are available to etch coated substrate 104. In other words, applying a DC voltage to coated substrate 104 in the presence of RF plasma will produce a far greater etch rate than applying the same voltage in the absence of RF plasma. Even though there are still sputtered atoms arriving at coated substrate 104, they are removed as quickly as they arrived by the combined effect of the dense plasma and high substrate voltage.

Alternatively, the deposition and etching steps may be sequential. If both steps are accomplished using sputtering, this may be accomplished by simply turning off the power to the deposition source of clusters 101 and turning on the power to substrate 103. Or alternatively, the deposition and etching steps may take place in separate chambers.

Although the present invention has been described in considerable detail with reference to certain preferred versions thereof, other versions are also possible. For example, an inhomogeneous surface can be created on structures other than implants to encourage cell attachment. Therefore, the spirit and scope of the appended claims should not be limited to the description of the preferred versions contained herein.

Thus, it is seen that the objects of the present invention are efficiently obtained, although modifications and changes to the invention should be readily apparent to those having ordinary skill in the art, which modifications are intended to be within the spirit and scope of the invention as claimed. It also is understood that the foregoing description is illustrative of the present invention and should not be considered as limiting. Therefore, other embodiments of the present invention are possible without departing from the spirit and scope of the present invention.

## Claims

1. A method of forming an inhomogeneous surface (106) comprising the steps of:

   - depositing a discontinuous coating (101) of atoms of a first substance on a substrate (103) comprising a second substance, said first and second substances having first and second etch rates, respectively, wherein said first etch rate is different than said second etch rate; and,
   - etching the substrate (103).

2. The method of Claim 1 wherein said step of etching said substrate (103) comprises etching said substrate (103) via physical sputtering.

3. The method of one of the preceding claims wherein said step of depositing said discontinuous coating (101) comprises depositing via physical vapor deposition.

4. The method of one of the preceding claims wherein said steps of depositing and etching are performed simultaneously.

5. The method of one of the preceding claims wherein said discontinuous coating of atoms forms a plurality of clusters (101), each of said plurality of clusters (101) having lateral dimensions from about ten nanometers to about one thousand nanometers.

6. The method of one of the preceding claims wherein said inhomogeneous surface comprises a plurality of structures (106), each of said structures (106) having heights from about ten nanometers to about ten thousand nanometers.

7. The method of one of the preceding claims wherein said discontinuous coating (101) is deposited on a mechanically rough portion of said substrate (103).

8. The method of one of the preceding claims wherein said first etch rate is less than said second etch rate.

9. The method of one of the preceding claims further comprising the step of:

   - applying a voltage to said substrate (103), wherein applying said voltage causes a formation of structures (101) on said substrate (103), each of said structures (101) having a height of about one hundred nanometers to about ten thousand nanometers.

10. The method of one of the preceding claims wherein said discontinuous coating of atoms forms a plurality of clusters (101) and dimensions of said plurality of clusters (101) are arranged to create a plurality of final structures that preferentially bind at least one biological structure having a specific size.

11. The method of one of the preceding claims further comprising the step of:

    - depositing a layer on said substrate (103), wherein said layer and said coating (101) have a first chemical binding energy, said layer and said substrate (103) have a second chemical binding energy, said first and second chemical binding energies are different, and said first and second chemical binding energies are arranged to alter nucleation characteristics of said coating (101).

12. The method of one of the preceding claims wherein said substrate (103) is a part of an orthopedic implant.

13. A method of preparing a substrate (103) for cell attachment comprising the steps of:

    - providing said substrate (103); and,
    - depositing a coating of atoms on said substrate (103) so that said atoms form clusters (101), each of said clusters (101) having a size and a distance from other of said clusters (101).

14. The method of Claim 13 wherein said size of each cluster (101) and said distance from other of said clusters (101) are chosen to preferentially bind at least one biological structure having a specific size.

15. The method of Claim 13 or 14 further comprising the step of:

    - changing a chemical binding energy between said substrate (103) and said coating of said atoms (101).

16. A method of creating a surface for enhancing cell growth on said surface, said surface comprising a first substance (103), said method comprising the steps of:

    - depositing a discontinuous coating of atoms (101) of a second substance on said surface (103), wherein said first and second substances (101, 103) have first and second sputter yields, respectively, and said first and second sputter yields are different; and
    - applying a voltage to said surface (103) to cause sputtering.

17. The method of Claim 16 wherein said steps of depositing of said coating (101) and applying of said voltage are performed simultaneously.

18. The method of Claim 16 or 17 wherein said step of applying said voltage is performed after said step of depositing said coating.

19. The method of one of Claims 16-18 wherein said surface (103) is a part of an orthopedic implant.

20. A substrate (103) arranged for cell attachment comprising:

- a surface; and
- a discontinuous coating of atoms on said surface, wherein said coating comprises a plurality of clusters of atoms (101), each of said plurality of clusters (101) having lateral dimensions from about ten nanometers to about one thousand nanometers.

104

101

103

Fig. 1

Fig. 2

**EP 1 826 293 A1**

## EUROPEAN SEARCH REPORT

**European Patent Office**

**Application Number**

EP 07 00 2850

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 399 238 A (KUMAR NALIN [US]) 21 March 1995 (1995-03-21) <br><br> * column 4, line 55 - column 7, line 50; claims; figures 1A-1D * <br> ----- | 1-3,5,6, 8-10, 13-16, 18,20 | INV. C23F4/00 A61F2/30 A61L27/06 |
| X | WO 97/26026 A (ETEX CORP [US]) 24 July 1997 (1997-07-24) <br><br> * page 13, line 21 - page 18, line 2; claims; figure 2 * <br> ----- | 1-4,7,8, 10, 12-17,19 | |
| X | US 2004/134886 A1 (WAGNER DONALD J [US] ET AL WAGNER DONALD J [US] ET AL) 15 July 2004 (2004-07-15) * paragraphs [0034] - [0046]; claims; figures 1-3 * <br> ----- | 1,7,8, 12-15 | |
| A | EP 1 440 669 A1 (DINKELACKER WOLFGANG DR MED DE [DE]) 28 July 2004 (2004-07-28) * paragraph [0028]; claims; figures 7,8 * <br> ----- | 1-20 | |
| | | | **TECHNICAL FIELDS SEARCHED (IPC)** |
| A | AIMI M F ET AL: "High-aspect-ratio bulk micromachining of titanium" NATURE MATERIALS NATURE PUBLISHING GROUP UK, vol. 3, no. 2, February 2004 (2004-02), pages 103-105, XP002434743 ISSN: 1476-1122 * the whole document * <br> ----- | 1-20 | C23F A61F A61L B81C |

The present search report has been drawn up for all claims

| Place of search | Date of completion of the search | Examiner |
|---|---|---|
| Munich | 23 May 2007 | Mauger, Jeremy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 00 2850

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2007

| Patent document cited in search report | | Publication date | Patent family member(s) | | Publication date |
|---|---|---|---|---|---|
| US 5399238 | A | 21-03-1995 | NONE | | |
| WO 9726026 | A | 24-07-1997 | AU | 725983 B2 | 26-10-2000 |
| | | | AU | 1835097 A | 11-08-1997 |
| | | | EP | 0880369 A2 | 02-12-1998 |
| | | | JP | 2000503571 T | 28-03-2000 |
| | | | NZ | 331008 A | 28-01-2000 |
| | | | US | 5843289 A | 01-12-1998 |
| | | | US | 6582470 B1 | 24-06-2003 |
| US 2004134886 | A1 | 15-07-2004 | AU | 2004204772 A1 | 29-07-2004 |
| | | | CA | 2512954 A1 | 29-07-2004 |
| | | | EP | 1583669 A2 | 12-10-2005 |
| | | | US | 6193762 B1 | 27-02-2001 |
| | | | US | 5507815 A | 16-04-1996 |
| | | | US | 5258098 A | 02-11-1993 |
| | | | US | 5922029 A | 13-07-1999 |
| | | | WO | 2004062477 A2 | 29-07-2004 |
| EP 1440669 | A1 | 28-07-2004 | AT | 345747 T | 15-12-2006 |
| | | | US | 2004153154 A1 | 05-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5282861 A
- US 5669909 A
- US 5984967 A
- US 6261322 B
- US 6645206 B
- US 6613091 B
- US 6375655 B